**Europäisches Patentamt**

**European Patent Office**

**Office européen des brevets**

(11) Veröffentlichungsnummer: **0 307 792**

**A2**

## (12) EUROPÄISCHE PATENTANMELDUNG

(21) Anmeldenummer: **88114664.1**

(22) Anmeldetag: **08.09.88**

(51) Int. Cl.4: **A61B 10/00**

(30) Priorität: **18.09.87 DE 3731407**

(43) Veröffentlichungstag der Anmeldung:
**22.03.89 Patentblatt 89/12**

(84) Benannte Vertragsstaaten:
**AT CH DE GB LI**

(71) Anmelder: **Schlüter, Gert**
**Im Höfle 22**
**D-7803 Gundelfingen(DE)**

(72) Erfinder: **Schlüter, Gert**
**Im Höfle 22**
**D-7803 Gundelfingen(DE)**

(74) Vertreter: **Patentanwälte Dipl.-Ing. Hans**
**Schmitt Dipl.-Ing. Wolfgang Maucher**
**Dreikönigstrasse 13**
**D-7800 Freiburg i.Br.(DE)**

(54) **Vorrichtung zum Gewinnen von Abstrichmaterial.**

(57) Eine Vorrichtung dient zum Gewinnen von Abstrichmaterial, insbesondere für die Zell- und Keimdiagnostik und hat einen Stiel (11), der an seinem Einführende (12) einen Abstrichkörper (13) aufweist. Gemäß der Erfindung ist der Abstrichkörper (13) im wesentlichen aus einem wenigstens einseitig am Stiel (11) radial vorstehenden, biegsamen Porenkörper (14) od. dgl. gebildet.

Mit einer solchen Abstrichvorrichtung (10) läßt sich unter weitestgehender Vermeidung von Verletzungsgefahr bei einfacher Handhabung eine vergleichsweise große und gut erhaltene Menge von Abstrichmaterial entnehmen und gut auf einen Objektträger übertragen.

Fig.2

## Vorrichtung zum Gewinnen von Abstrichmaterial

Die Erfindung betrifft eine Vorrichtung gemäß dem Oberbegriff von Anspruch 1.

Mit einer solchen Vorrichtung soll für die allgemeine Zell- und Keimdiagnostik von Organoberflächen, aus Organvertiefungen (Krypten) sowie insbesondere aus engen oder weiten Körperhöhlen Untersuchungsmaterial herausgefördert und auf Objektträger deponiert oder in Nährmedien übertragen werden können.

Man kennt bereits etliche für diese Untersuchungen geschaffene Abstrichvorrichtungen, die jedoch noch wesentliche Nachteile aufweisen.

Nicht mehr neu ist z. B. ein Abstrichtupfer, der an einem Stiel einen Abstrichkörper aus Fasermaterialien wie z. B. aus Baumwolle, aus Zellulose-Zwei-Eins/Zwei-Acetat, aus Äthylzellulose oder aus Alginat aufweist. Mit diesen Abstrichvorrichtungen soll bei menschlichen Organen, z. B. im gynäkologischen Bereich von der Portio-Oberfläche oder aus dem Zervikal-Kanal, Zellmaterial (oder Keime) abgestrichen oder abgeschabt und der weiteren Untersuchung zugeführt werden. Das bedeutet, daß das mit der vorerwähnten Abstrichvorrichtung abgeschabte Material, welches an deren Abstrichkörper haftet, von dieser Vorrichtung wiedergewonnen werden muß. Für die Zelldiagnostik erfolgt die Wiedergewinnung direkt durch einen Ausrollvorgang des Abstrichkörpers auf einen Objektträger; (das sind in der Regel "Glasplättchen"). An die Übertragung auf den Objektträger schließen sich dann spezielle, bekannte Präparationsschritte an. Ein gynäkologischer Abstrich wird gewöhnlich in Alkohol fixiert und danach einem Färbeverfahren, z. B. nach Papanicolaou unterzogen, um die Zellen für die mikroskopische Beurteilung sichtbar zu machen, wobei es insbesondere darauf ankommt, die feinmorphologischen Strukturen in Kernen und Zytoplasma darzustellen.

Zell- und Keimgewinnungstechniken stellen besonders im Hinblick auf die diagnostische Aussagekraft der Präparate (Krebsfrüherkennung oder Keim-und Erregerdiagnostik) hohe Anforderungen an die Entnahmetechnik und an die Präparation. Ein Entnahmeinstrument für die Abstrichmaterial-Gewinnung (Zellgewinnung) muß von seiner Ausbildung her bei der Anwendung eine hoch-effiziente Wirkungsweise haben, ohne traumatisch oder zellschädigend zu wirken. Das bedeutet, daß das Instrument an die Anatomie des abzustreichenden Organs angepaßt oder anpaßbar sein muß, z. B., um auch aus Krypten unterschiedlicher Lage und Tiefe optimal Material fördern zu können.

Die vorbekannte Abstrichvorrichtung mit einem Abstrichkörper (Tupfer) aus Baumwollwatte hat in der praktischen Anwendung gravierende Nachteile hinsichtlich der Zellgewinnung vom Organ und der Zellwiedergewinnung auf den Objektträger (bgl. G. Schlüter et al "Exaktere Zellfix-Zytologie mit neuer Abstrich- und Präparationstechnik" in "Diagnostik 17" (1984) Nr. 5, S. 27 - 29 - MMV Medizin verlag, D-8000 München 80). Bei solchen Abstrichvorrichtungen wird der Abstrichkörper dadurch hergestellt, daß in üblicher Weise Baumwollwatte als Tupfer auf das Ende eines Stiels aufgewickelt wird. Wie Versuche gezeigt haben, bleibt ein verlgeichsweise hoher Prozentsatz der abgestrichenen Zellen unlöslich mit dem Faserbündel des Abstrichkörpers verbunden und geht für die Untersuchung verloren. Das führte zu einer Erhöhung der Falsch-Negativ-Rate bei der gynokologischen Krebsfrühdiagnose.

Man hat diesen Nachteil bereits dadurch zu vermindern gesucht, daß man eine verbesserte Abstrichvorrichtung geschaffen hat, bei welcher der Abstrichkörper einen aus Ethylzellulose hergestellten Fasertupfer aufweist; (vg. DE-PS 25 13 941 u. DE-PS 25 59 510). Solche Abstrichvorrichtungen erweisen sich deutlich als ergiebiger in der Gesamtzellausbeute, wenn die Fasern bei der Zellwiedergewinnung in Alkohol gelöst werden. Als gravierender Nachteil hat sich jedoch erwiesen, daß die Herstellung der speziellen, alkohollöslichen Fasern vergleichsweise teuer ist, insbesondere wenn man beachtet, daß die Gesamtmenge, die z. B. für ein bestimmtes Marktgebiet benötigt wird, auf industrielle Verhältnisse bezogen, klein ist. U. a. haben sich diese Abstrichvorrichtungen deshalb praktisch nicht einführen können.

Man hat für die Durchführung von Abstrichen im Zervikal-Bereich auch bereits eine Zellbürste empfohlen, die nach der Art einer Flaschenbürste hergestellt ist und bei ihrer Anwendung das Zellmaterial von der Organwand abbürsten soll. Bei dieser Zellbürste werden die einzelnen Borstenbüschel von zwei gewendelten Metalldrähten gehalten, die notwendigerweise etwas über das vorderste Borstenbüschel vorstehen. Selbst wenn man dieses vorstehende Doppeldraht-Ende verrundet, stellt es immer noch eine für den Eingriff gefährliche, über den Borstenbereich vorstehende Spitze dar, die leicht zu Verletzungen führen kann. Dies gilt besonders dann, wenn die Zellbürste schräg eingeführt werden muß oder ungeschickt gehandhabt wird.

Ferner hat man auch bereits als Abstrichvorrichtung einen Spatel in harter Ausbildung geschaffen. Dieser hat u. a. den Nachteil, daß beim Einführen und Abstreichen leicht Gewebeläsionen entstehen, die zu Blutungen führen. Ein auf diese Weise entnommenes Zellmaterial wird in aller Regel stark mit Blutzellen durchmischt sein, welches das für die Diagnostik relevante Zellmaterial prominent bedekken und somit z. B. das Auffinden von Krebszellen erschweren oder gar unmöglich machen kann. Hart ausgebildete Abstrichinstrumente wie der vorerwähnte Spatel erzeugen außerdem vermehrt Zellartefakte und deponieren das Material uneinheitlich (zu dick oder zu dünn) auf den Objektträger. Alle vorbeschriebenen Instrumente bedürfen einer geschickten Führung durch den Anwender, wenn sie effektiv Untersuchungsmaterial fördern sollen. Da in manchen Organregionen, z. B. im Zervixkanal oder im Uteruskavum, nicht unter Sicht gearbeitet werden kann, wird die Abstrichmanipulation bzw. der Kontakt zum Organgewebe, zur Haut oder Schleimhaut, insbesondere auch das Erreichen von Organvertiefungen, vom Zufall mitbeeinflußt. In der Praxis bedeutet das, daß frühe maligne Veränderungen, wenn diese in Vertiefungen der auskleidenden Schleimhaut von Organhöhlen angesiedelt sind, von den ausgeführten Instrumenten nicht oder jedenfalls nicht mit der erwünschten Sicherheit erfaßt werden. Das hat zur Folge, daß die Krebsfrüherkennung negativ belastet wird. Es gibt bis heute keine Früherkennung des Endometriumkarzinoms, weil man mit dem derzeit zur Verfügung stehenden Instrumenten die auskleidende Schleimhaut der Uterushöhle nicht raum greifend und lückenlos abstreichen kann; (Endometrium = auskleidende Schleimhaut der Gebärmutter).

Man kennt für die Probengewinnung aus dem Uterus auch bereits Spül- und Aspirationsverfahren. Diese haben sich jedoch nicht bewährt, weil der Abschilferungsgrad des Endometriums (Schleimhaut der Uteruswand) hormonell gesteuert wird. Diese Verfahren fördern daher nur Zellen, die sich in lockeren Verbänden von der Schleimhaut lösen lassen.

Aufgabe der vorliegenden Erfindung ist daher insbesondere die Schaffung einer Vorrichtung zum Gewinnen von Abstrichmaterial, bei welcher die Nachteile der vorbekannten Abstrichvorrichtungen zumindest weitgehend vermieden werden; insbesondere soll eine Vorrichtung geschaffen werden, mit der bei einfacher Handhabung ein gut erhaltenes, repräsentatives und von der Menge her für die Diagnostik ausreichendes Zellmaterial gewonnen und gut auf den Objektträger übertragen werden kann.

Die erfindungsgemäße Lösung dieser Aufgabe besteht bei einer Vorrichtung gemäß dem Oberbegriff von Anspruch 1 insbesondere in den Kennzeichnungsmerkmalen des 1. Anspruches.

Durch diese spezielle Ausbildung der Vorrichtung wird eine möglichst gute Anpassung an die Anatomie der zu untersuchenden Organe erreicht, so daß eine zellfördernde Wirkung sowohl auf glatten als auch auf zerklüfteten Organflächen als auch in Körperhöhlen unterschiedlicher Lumina mit genügend gleichmäßigem Ergebnis möglich ist.

Eine vorteilhafte Weiterbildung der Erfindung besteht darin, daß der Abstrichkörper als vorzugsweise wenigstens etwa mittig am Einführende des Stiels angebrachte poröse Scheibe ausgebildet ist. Sie kann z. B. einen Durchmeser von etwa 10 mm und eine Dicke von etwa 3 mm haben. Ein solcher biegsamer Porenkörper kann sich gut der speziellen Umrißform auch engerer Körperhöhlungen anpassen und er ist gut geeignet, vergleichsweise viel Zellmaterial aufzunehmen und herauszutransportieren. Beim Einschieben in eine Körperhöhlung kann sich nämlich die zum Einführende der Vorrichtung gewandte Oberseite der Scheibe gut an die Wandung des Körperkanal-Abschnittes anlegen, während beim Zurückziehen der Vorrichtung sich zusätzlich die Rückseite der Scheibe an diese Wandung anlegen kann. Es wird deshalb eine Vielzahl von Poren angeboten, in die Zellmaterial aufgenommen und abtransportiert werden kann.

An der Rückseite einer solchen porösen Scheibe kann in Weiterbildung der Erfindung auch eine biegesteife Widerlagerscheibe am Stiel der Vorrichtung vorgesehen sein. In diesem Fall eignet sich die Vorrichtung besonders gut zur Entnahme von Zellmaterial od. dgl. an Stellen, die etwas unter Druck abgetupft werden sollen.

Für besondere Anwendungsfälle hat sich als besonders vorteilhaft erwiesen, wenn der Abstrichkörper als fahnenartiger Streifen ausgebildet und am Einführende des Stiels der Vorrichtung angebracht ist. Eine besonders vorteilhafte Ausführung der erfindungsgemäßen Vorrichtung besteht darin, daß der Abstrichkörper aus einer Polyurethan-Schaumschicht od. dgl. körperfreundlicher, verfahrensneutraler schwammartiger Schaumschicht besteht. Dabei ist besondere zweckmäßig, wenn diese Polyurethanschaumschicht etwa 600 bis 2.500 Poren / cm² aufweist, vorzugsweise etwa 1.500 Poren / cm².

Versuche haben folgendes gezeigt : Solche Porenkörper aus diesem Kunststoff sind einerseits körperfreundlich, sie sind bei den üblichen Abmessungen der Abstrichkörper weich und anpassungsfähig, gleichzeitig nehmen ihre Poren das Zellmaterial od. dgl. gut auf ohne praktisch ins Gewicht fallenden Verletzungsgefahr oder Gefahr des Entstehens von Artefakte zu bilden, zum anderen ist dieser Werkstoff für die weitere Durchführung des Verfahrens verfahrensneutral und die Poren geben das Zellmaterial gut an den Objektträger ab. Die vorerwähnte, vorteilhafte Porenhäufigkeit pro cm² begünstigt dabei die Entnahme von vergleichsweise umfangreichen Mengen von Zellmaterial od. dgl.

Zusätzliche Weiterbildungen der Erfindung sind in weiteren Unteransprüchen aufgeführt.

Nachstehend wird die Erfindung anhand von mehreren Ausführungsbeispielen in Verbindung mit der Zeichnung mit den erfindungswesentlichen Einzelheiten noch näher erläutert. Es zeigt :

Fig. 1 stark schematisiert einen Längsschnitt durch eine Gebärmutter und dem inneren Endbereich der Scheide,

Fig. 2 eine Vorrichtung zum Gewinnen von Abstrichmaterial, bei welcher der Abstrichkörper als poröse Scheibe ausgebildet ist,

Fig. 3 eine Stirnansicht der porösen Scheibe gemäß Fig. 2,

Fig. 4 eine Vorrichtung gemäß Fig. 2 u. 3 beim Einschieben in eine sich verengende Körperhöhlung,

Fig. 5 die Vorrichtung nach Fig. 2 u. 3 beim Herausziehen aus einer Körperhöhlung,

Fig. 6 die Stirnansicht eines etwa scheibenförmigen Abstrichkörpers, der in Segmente unterteilt ist,

Fig. 7 eine Vorrichtung, deren Abstrichkörper als fahnenartiger Streifen ausgebildet ist und

Fig. 8 eine gegenüber Fig. 2 abgewandelte Vorrichtung.

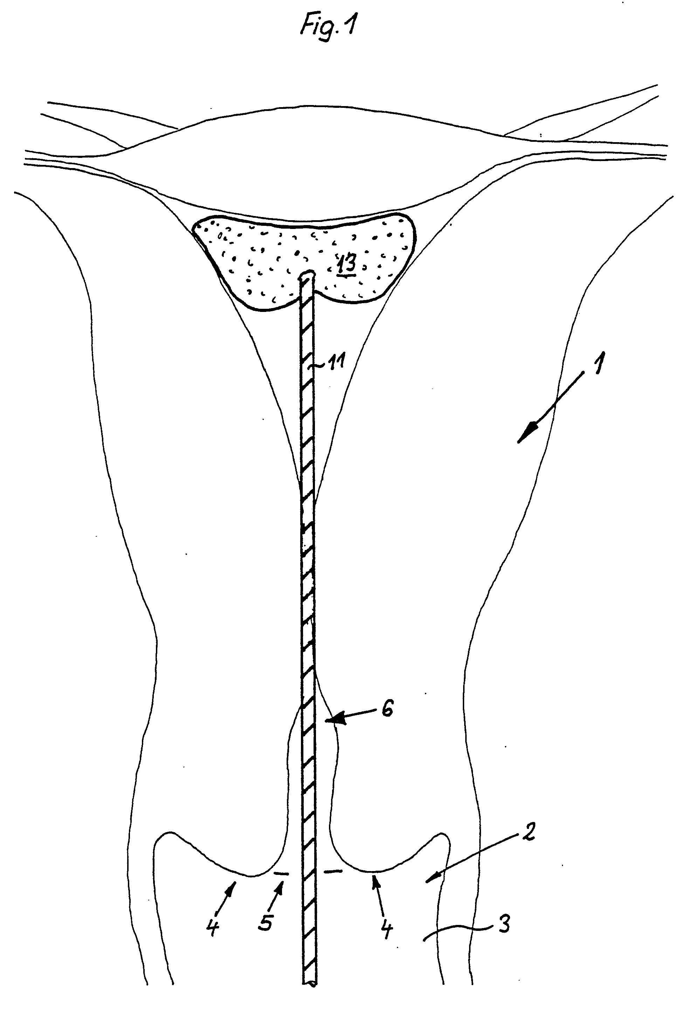

Fig. 1 zeigt einen stark schematisierten Längsschnitt durch eine im ganzen mit 1 bezeichnete Gebärmutter und dem inneren Ende 2 der Scheide 3. Mit 4 sind das Scheidengwölbe, mit 5 die Portio vaginalis und mit 6 der Zervixkanal bezeichnet.

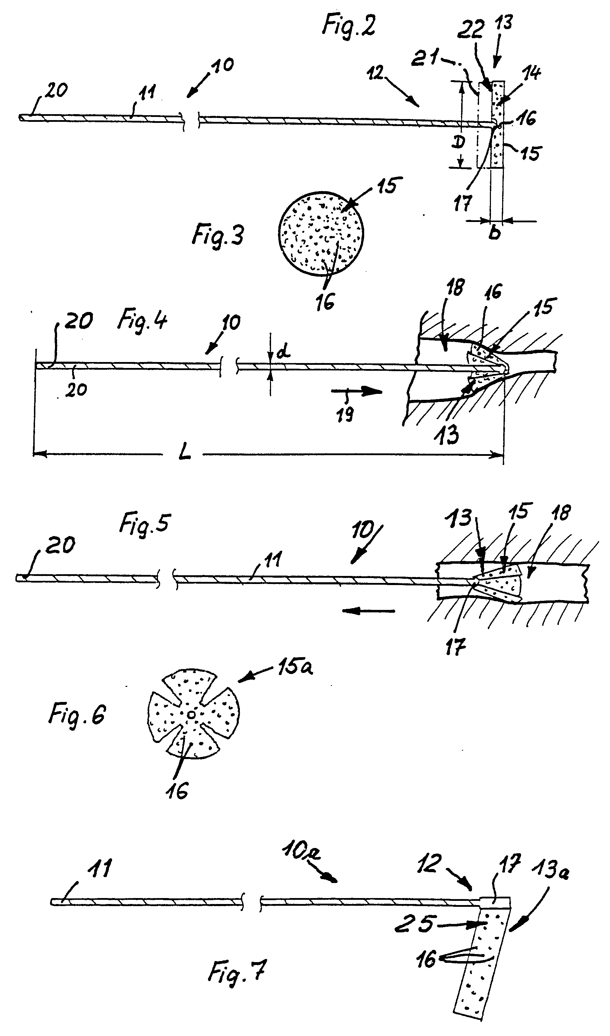

Fig. 2 zeigt nun eine im ganzen mit 10 bezeichnete Vorrichtung zum Gewinnen von Abstrichmaterial, das insbesondere für die Zell- und Keimdiagnostik dient. Diese Abstrichvorrichtung 10 hat in bekannter Weise einen dünnen Stiel 11, an dessen einem Ende, nämlich dem Einsteckende 12, ist ein Abstrichkörper 13 vorgesehen. Beim erfindungsgemäßen Abstrichkörper 10 besteht nun dieser im wesentlichen aus einem wenigstens einseitig radial am Stiel 11 vorstehenden, biegsamen Porenkörper 14. Im Ausführungsbeispiel nach Fig. 2 u. 3 ist dabei der mit zahlreichen Poren 16 versehene Abstrichkörper 14 als poröse Kreisscheibe 15 ausgebildet. Sie ist etwa mittig am Einführende 12 des Stiels 11 befestigt und hat z. B. einen Durchmesser D von etwa 10 mm und eine Dicke b von 3 mm. Als besonders vorteilhaft hat sich dabei ein Abstrichkörper 13 herausgestellt, der aus einer Polyurethanschaumschicht od. dgl. körperfreundlicher, verfahrensneutraler schwammartiger Schaumschicht besteht, wie er z. B. schematisiert mit den Poren 16 gut aus Fig. 1 bis 7 erkennbar ist. Versuche haben gezeigt, daß sich Polyurethanschäume mit einer Porösität von 650 Poren/ cm$^2$ bis 2.500 Poren / cm$^2$, insbesondere Schäume mit einer Porösität von etwa 1500 Poren / cm$^2$ aus Polyurethan-Schaum als besonders gut erwiesen. Fig. 2 sowie Fig. 4 bis 6 zeigen noch, daß die Kreisscheiben 15 in ihrem Zentrum auf die Applikationsstiele 11, die aus Holz oder aus PVC bestehen können, befestigt sind, was zweckmäßigerweise mit Hilfe eines Acrylatklebers 17 erfolgt. Diese Stiele 11 haben gewöhnlich eine Länge L von 160 mm und einen Durchmesser d von 2,5 mm (vgl. Fig. 4). Bei der vorzugsweise gewählten Dicke b von etwa 3 mm und der porösen Ausbildung von etwa 1.500 Poren / cm$^2$ zeigen die Kreisscheiben 15 aus Polyurethanschaum eine hohe Seitenflexibilität. Wird beispielsweise eine Abstrichvorrichtung 10 gemäß Fig. 2 in eine Körperhöhlung 18 von geringerer lichter Weite als es dem Durchmesser D der Kreisscheibe 15 entspricht, entsprechend dem Pfeil 19 (Fig. 4) eingeführt, klappt die Kreisscheibe 15 durch von oben und/oder von der Seite her wirkenden Druck zum äußeren Ende des Stiels hin um. Dabei können sich Teile der Kreisscheibe 15 gut einseitig oder zirkulär gegen den Stiel 11 abstützen und die Kreisscheibe 15 paßt sich unter Faltenbildung an das Lumen der Körperhöhlung 18 an. Wird die Abstrichvorrichtung 10 durch eine solche Körperhöhlung 18 zunächst im Sinne des Pfeiles 19 (Fig. 4) durchgeschoben, kann sich die eingeengte Kreisscheibe 15 wieder etwa in die ursprüngliche Scheibenform (Fig. 2) entfalten. Wird anschließend die Abstrichvorrichtung 10 durch die vorerwähnte Körperhöhlung 18 zurückgezogen, faltet sich die Kreisscheibe 15 von ihrer Befestigungsstelle am Stiel 11 weg kelchartig ein, wie es gut aus Fig .5 erkennbar ist. Dann kann die in der Ausgangsposition (Fig. 2) dem äußeren Ende 10 des Stiels 11 zugewandte rückwärtige Seite der Scheibe 15 sich an die Innenwand der Höhlung 19 anpassen und entsprechendes Ab strichmaterial mitnehmen. Die vorbeschriebene Arbeitsweise wird nachstehend beispielhaft bei der Anwendung am Zervixkanal 6 beschrieben. Da der Gebärmutterhalskanal (Endozervix) eng ausgebildet sein kann, wird die Abstrichvorrichtung 10 mit einer Kreisscheibe 15 mit einem Durchmesser D von 10 mm durchgeführt. Unter drehender Bewegung wird die Abstrichvorrichtung in den Zervixkanal 6 eingeführt. Hierbei legt sich die aus Polyurethanschaum bestehende Kreisscheibe 15, wie in Verbindung mit Fig. 4 vorstehend beschrieben, mit ihrer Rückseite einerseits an den Stiel 11 an und paßt sich mit ihrer Vorderseite der Ausbildung des Gebärmutterhalskanals an. Die schirmartig zusammengefaltete Kreisscheibe 15 kann sich dabei in Abhängigkeit der Ausbildung des Kanals engen und weiten. Wenn die Abstrichvorrichtung 10 etwa im ersten Drittel des Zervixkanals 6 (also im Endozervixbereich) plaziert ist, wird der Stiel 11 gedreht, wobei dann die Poren 16 der Polyurethanschaum-Kreisscheibe 15 an der Körperwandung reiben und Zellmaterial aufnehmen. Danach wird die Abstrichvorrichtung 10 zurückgezogen. Die Kreisscheibe 15 stülpt sich dabei nach oben um, wie es Fig. 5 zeigt, so daß sich beim Herausziehen auch die Unterseite der Kreisscheibe 15 mit Zellmaterial aus der gesamten kontaktierten Länge des Zervixkanals 6 belegt. Danach erfolgt die Zellübertragung von der Kreisscheibe 15 auf die für die Untersuchung vorgesehenen Objektträger, also in der Regel auf entsprechende Glasplättchen,

wobei die Ober- bzw. Unterseite der Kreisscheibe auf dem vorher mit einer alkoholischen Lösung benetzten Objektträger abgerollt wird, was bequem durch Drehen des Stieles 11 durchzuführen ist. Bei diesem Vorgang wird die zelltragende und mit Alkohol gesättigte Kreisscheibe 15 gegen den Stiel 11 gedrückt und das Zellmaterial spült sich aus den Poren 16 aus und deponiert sich im fixierten (konservierten) Zustand auf dem Objektträger.

Die Abstrichvorrichtung 10 ist zum einmaligen Gebrauch bestimmt und wird nach diesem Vorgang verworfen. Die vorstehend beschriebene Entnahme von Abstrichmaterial aus dem Zervixkanal kann analog auch bei anderen Körperhöhlen erfolgen.

Eine etwas abgewandelte Anwendungsmöglichkeit besteht darin, daß mit der Abstrichvorrichtung 10 Abstrichmaterial von einer glatten Organfläche, z. B. von der Haut oder Schleimhaut gewonnen werden soll. Dann wird unter Handhabung des Applikationsstieles 11 die vorzugsweise aus Polyurethanschaum bestehende Kreisscheibe 15 auf die Organfläche aufgesetzt und mehrfach um die Längsachse des Stieles 11 gedreht. Dabei wird Zellmaterial abgerieben und auf die Kreisscheibe 15 und in deren Poren 16 übertragen. Wenn es darauf ankommt, daß bei diesem Vorgang ein gewisser Druck auf die Haut oder Schleimhaut ausgeübt werden soll, versieht man zweckmäßigerweise die Abstrichvorrichtung 10 an der Rückseite 22 der Kreisscheibe 15 mit einer im wesentlichen biegesteifen Widerlagerscheibe 21, wie sie in Fig. 2 strichpunktiert dargestellt ist, Diese Widerlagerscheibe besteht zweckmäßigerweise aus Kunststoff, kann bedarfsweise vom äußeren Ende 20 des Stiels 11 her auf diesen bis zur Rückseite 22 der kreisscheibe 15 aufgeschoben werden. In dieser Stellung wird sie durch Reibschluß am Stiel 11 klemmend festgehalten. Diese Widerlagerscheibe 21 erlaubt insbesondere bei Kreisscheiben 15 mit größerem Durchmesser, z. B. D = 20 mm, wie dies in Fig. 2 und 3 dargestellt ist, insbesondere eine Kreisscheibe 15 auf eine Organfläche unter einem bestimmten Druck aufzusetzen, dabei zu drehen und Zellmaterial abzunehmen. Die Zellübertragung von der Kreisscheibe 15 auf den Objektträger erfolgt in der Weise, wie dies beim bereits beschriebenen Beispiel der Handhabungen der Abstrichvorrichtung bei der Anwendung im Zervixkanal 6 erwähnt wurde. Gegebenenfalls kann beim Abrollen der Kreisscheibe 15 auf dem Objektträger die Widerlager-scheibe 21 vorher von der Kreisscheibe 15 zurückgezogen bzw. ganz vom Stiel 11 entfernt werden.

Ein anderes Anwendungsbeispiel ist die Entnahme von Abstrichmaterial auf Organflächen mit Vertiefungen, z. B. bei der Portio vaginalis 5 . Eine Abstrichvorrichtung 10, z. B. mit einem Kreisscheiben-Durchmesser D = 20 mm, jedoch ohne Widerlagerscheibe 21, wird unter leichtem Druck auf die Portiofläche aufgesetzt, wobei der Stiel 11 auf den Gebärmuttermund gerichtet ist. Da dieser vertieft ausgebildet ist, nimmt die poröse, leicht biegsame Kreisscheibe 15 aus Polyurethanschaum eine leichte Kegelform an. Sie kann sich den unterschiedlichen, jeweils anatomisch vorliegenden Vertiefungen der Portio vaginalis 5 anpassen. Deren Oberfläche wird unter drehender Bewegung der Kreisscheibe 15 abgestrichen. Die Zellübertragung von der Kreisscheibe 15 auf den Objektträger erfolgt dann in der bereits erwähnten Weise.

Für besondere Anwendungsaufgaben wie z. B. für die Zellgewinnung, aus tiefergelegenen Körperhöhlen, die nur endoskopisch erreichbar sind, wie z. B. Magen, Darm oder Endometrium, wird die Abstrichvorrichtung 10 ähnlich der im Zusammenhand mit Fig. 4 u. 5 beschriebenen Arbeitsweise in einem Rohr (Endoskop) zusammengefaltet untergebracht. Erst nachdem dieses Applikationsrohr die zu untersuchende Organhöhle erreicht hat, wird der Abstrichkörper 13 der erfindungsgemäßen Abstrichvorrichtung 10 aus dem Endoskop ausgeschoben. Dann kann entweder blind (in kleinen Höhlen wie z. B. dem Uterus) oder unter endoskopischer Sicht die Zellgewinnung erfolgen. Nach dem Abstrichvorgang wird die poröse Kreisscheibe 15 in das Applikationsrohr zurückgezogen und zusammen mit diesem aus der Organhöhle entfernt. Die Zellgewinnung aus dem Abstrichkörper 13 erfolgt wieder in der bereits beschriebenen Weise.

Die vorteilhafte Arbeitsweise der erfindungsgemäßen Abstrichvorrichtung 10 wurde in einem Vergleichsversuch getestet. Dabei wurde von einem infizierten Wundgebiet der Haut mit Hilfe einer Abstrichvorrichtung 10, die mit einer Kreisscheibe 15 und einer Widerlagerscheibe 21 zu deren Stabilisierung ein Keimgewinnungsnachweis durchgeführt. Dieser wurde mit einem Keimgewinnungsnachweis unter Verwendung eines Wattetupfers verglichen. Die erfindungsgemäße Abstrichvorrichtung 10 zeigte eine sehr gute Aufnahmefähigkeit für das Sekret und erwies sich auch hinsichtlich der Abriebfähigkeit als besonders geeignet. Die Keimübertragung erfolgte in ein Nährmedium, in dem die Polyurethan-Kreisscheibe 15 ausgespült wurde. Im Gegensatz zum Test mit dem Wattetupfer wurde nicht nur eine höhere Keimzahl festgestellt, sondern bei dem Versuch mit der erfindungsgemäßen Vorrichtung 10 konnten außerdem staph. aureus nachgewiesen werden, die beim Wattetupfer-Ausstrich fehlten.

Die vorstehend beschriebenen Anwendungsfälle wurden mit der erfindungsgemäßen Vorrichtung 10 unter praxisbezogenen Bedingungen experimentell untersucht. Gegenüber konventionellen bzw. praxisüblichen Verfahren konnte hinsichtlich der diagnostischen Aussagekraft eine deutliche Verbesserung ermittelt werden. Es wurde nicht nur mehr Zellmaterial gefördert, sondern auch die sonst häufig auftretenden Präparationsartefakte wurden weitestgehend verhindert.

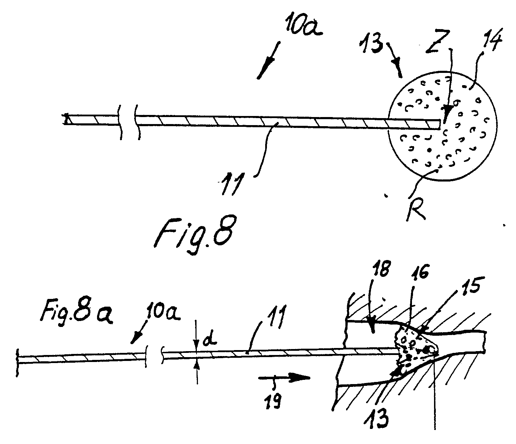

Die den Abstrichkörper bildende Scheibe muß nicht zwingend kreisförmig sein, obgleich eine Kreisscheibe 15 eine bevorzugte Ausführungsform ist. Eine etwas abgewandelte Form zur Kreisscheibe 15 stellt die rosettenförmige, etwa in vier gleichgroße Segmente unterteilte Scheibe 15 a gemäß Fig. 6 dar. Bei ihr ist die Anpassungsfähigkeit besonders bei sehr engen Körperhöhlungen 18, besonders gut.

Bedarfsweise kann der Abstrichkörper 13 erfindungsgemäß auch eine zylinderische oder olivenförmige Umrißform aufweisen. Als besonders vorteilhaft hat sich bei speziellen Anwendungen herausgestellt, wenn der Abstrichkörper 13 a als fahnenartiger Streifen 25 ausgebildet ist. Eine Abstrichvorrichtung 10, deren Abstrichkörper 13 a als fahnenartiger Streifen 25 ausgebildet ist, hat sich insbesondere für die Zell- und Keimgewinnung aus extrem engen Körperhöhlen wie z. B. aus dem Zervixkanal 6 oder aus der Harnröhre erwiesen. Der fahnenartige Streifen kann z. B. Abmessungen von 8 mm x 40 mm haben. Er wird, wie gut bei Fig. 7 erkennbar, am Einsteckende 12 eines Stiels 11 befestigt derart, daß der Streifen 25 in der Ausgangsstellung fahnenartig vom Stiel 11 absteht, wie in Fig. 7 erkennbar. Bei der Anwendung wird eine solche, abgewandelte Vorrichtung 10 a unter drehender Bewegung z. B. im Uhrzeigersinn in eine enge Körperhöhle 18 geschoben. Dabei dreht sich der zunächst fahnenartig abstehende Streifen 25 schraubenlinienförmig auf den Stiel 11 auf und legt sich daran eng an. So ist es besonders gut möglich, daß die verhältnismäßig große Fläche in ein enges Lumen einer Körperhöhlung 18 eingeführt werden kann. Bereits beim Einführen können Zellen und/oder Keime vom porösen Streifen 25 aufgenommen werden. Es hat sich herausgestellt, daß eine besonders gute Zell- und Keimgewinnung bei einer solchen Vorrichtung 10 dann erfolgt, wenn diese dann im Gegendrehsinn, also z. B. gegen den Uhrzeigersinn gedreht wird, wobei sich der auf dem Stiel 11 aufgewickelte fahnenartige Streifen 25 abwickelt und dabei einen reibenden Kontakt zur Organwand entfaltet. Hierbei wird umlaufend bzw. zirkulär eine große Menge Untersuchungsmaterial gewonnen und auf den fahnenartigen Streifen 25 übertragen. Beim Herausziehen -auch dieser Vorgang erfolgt drehend- wickelt sich der fahnenartige Streifen 25 wieder auf den Stiel 11 auf. Das Abstrichmaterial wird dann durch Abdrehen bzw. durch Abrollen unter festem Gegendruck auf den Objektträger übertragen oder das Abstrichmaterial wird in speziellen Flüssigkeiten vom Entnahmekörper abgespült.

Als Werkstoff für den Abstrichkörper 13 kommt nicht nur Polyurethan-Schaumstoff in Frage, sondern auch andere körperfreundliche und verfahrenneutrale Kunststoffschäume, z. B. Silidonschaum. Auch sind die Werkstoffe nicht auf Kunststoffschäume begrenzt; es könnten z. B. auch islandmoosartige, schwammig-poröse Werkstoffe herangezogen werden, welche körperfreundlich und verfahrensneutral sind. In der Praxis haben sich bisher jedoch Polyurethan-Schaumstoffe, insbesondere mit der bereits beschriebenen Porenverteilung, als besonders vorteilhaft, biokompatibel und verfahrensneutral erwiesen. Auch können Abstrichkörper 13 aus Polyurethan-Schaumstoff sich einerseits gut an die unterschiedlichen Formen des zu untersuchenden Körperteiles anpassen, ohne Verletzungen zu verursachen, andererseits können die Poren 16 bei diesem Werkstoff die abzustreichenden Zellen besonders gut aufnehmen und später in entsprechend großem Umfang wieder abgeben.

Zum Befestigen des Abstrichkörpers 13 aus Polyurethan-Schaumstoff hat sich ein Acrylatkleber als besonders vorteilhaft erwiesen. Acrylatkleber ist gewebefreundlich und verfahrensinert. Als Kleber kommen auch andere gewebe freundliche und verfahrensinerte Werkstoffe in Frage, z. b. Polysterol-Kleber. Auch hier hat sich Acrylatkleber als besonders vorteilhaft erwiesen.

Eine etwas abgewandelte Ausführung der Abstrichvorrichtung kann darin bestehen, daß der Abstrichkörper 13 nicht aus einem Porenkörper, sondern aus einem strukturierten, z. B. gewebten Vlies besteht. Bei einem solchen Vlies ersetzt die Vliesstruktur die Porung 16 beim Porenkörper 14. Die Feinheit der Maschen und die Durchmesser Kett- und Schußfäden beeinflussen dann die Oberflächenstruktur des Abstrichkörpers. Eine besonders vorteilhafte Anwendung eines aus Vlies bestehenden Abstrichkörpers besteht darin, daß der fahnenartige Streifen (25) aus Vlies besteht. Er kann z. B. besonders reißsicher ausgebildet sein.

Die Fig. 8 zeigt eine im ganzen mit 10b bezeichnete, gegenüber der Vorrichtung 10 nach Fig. 2 etwas abgewandelte Vorrichtung. Bei dieser Ausführung ist der Stiel 11 parallel zur Rückseite R des biegsamen Abstrichkörpers 13, der vorzugsweise aus einem Porenkörper 14 besteht, angeordnet. Dabei reicht der Stiel 11 vorzugsweise etwa bis zum Zentrum Z des im Seitenquerschnitt runden Abstrichkörpers 13 bzw. Porenkörpers 14 und der Stiel ist an der Rückseite dieser im Umriß kreisförmigen, in der Seitenansicht flachen Porenscheibe 14 beispielsweise mit Hilfe von einem Acyrlatkleber befestigt. Bei einer solchen Ausbildung ergeben sich folgende Vorteile : Es ist eine verhältnismäßig großflächige Verbindung zwischen dem Stiel 11 und dem Porenkörper 14 möglich und außerdem ist beim Durchführen des Abstriches, der gewöhnlich dadurch erfolgt, daß eine drehende und vorschiebende Bewegung durchgeführt wird, eine besonders gute Führung des Porenkörpers 14 gegeben (vgl. Fig.8a). Außerdem ist die Verbindung zwischem dem Porenkörper 14 od. dgl. und dem Stiel 11 herstellungstechnisch besonders einfach.

## Ansprüche

1. Vorrichtung (10) zum Gewinnen von Abstrichmaterial, insbesondere für die Zell- und Keimdiagnostik, mit einem Stiel (11), der an einem Einführende einen Abstrichkörper (13) aufweist, **dadurch gekennzeichnet** , daß der Abstrichkörper (13) im wesentlichen aus einem wenigstens einseitig radial am Stiel (11) vorstehenden, biegsamen Porenkörper (14) od. dgl. besteht.

2. Vorrichtung nach Anspruch 1, dadurch gekennzeichnet, daß der Abstrichkörper (13) als vorzugsweise wenigstens etwa mittig am Einführende (12) des Stiels (11) angebrachte poröse Scheibe, vorzugsweise Kreisscheibe (15) ausgebildet ist, z. B. mit einem Durchmesser von etwa 10 mm und einer Dicke von etwa 3 mm.

3. Vorrichtung nach Anspruch 1 oder 2, dadurch gekennzeichnet, daß an der Rückseite (22) der porösen Scheibe (15) eine biegesteife Widerlagerscheibe (21) anbringbar ist, die vorzugsweise auf den Stiel (11) aufschiebbar sowie an der Rückseite (22) des Abstrichkörpers (13) am Stiel (11) klemmend festlegbar ist.

4. Vorrichtung nach Anspruch 3, dadurch gekennzeichnet, daß die poröse Scheibe (15 a) in Segmente unterteilt ist.

5. Vorrichtung nach Anspruch 1, dadurch gekennzeichnet, daß der Abstrichkörper (13 a) als fahnenartiger Streifen (25) ausgebildet ist.

6. Vorrichtung nach einem oder mehreren der Ansprüche 1 bis 5, dadurch gekennzeichnet, daß der Abstrichkörper (13) aus einer Polyurethanschaumschicht od. dgl. körperfreundlicher, verfahrensneutraler schwammartiger Schaumschicht besteht.

7. Vorrichtung nach Anspruch 6, dadurch gekennzeichnet, daß die Polyurethanschaumschicht etwa 650 bis 2500 Poren / cm² aufweist, vorzugsweise etwa 1500 Poren / cm².

8. Vorrichtung nach einem oder mehreren der Ansprüche 1 bis 5, dadurch gekennzeichnet, daß der Abstrichkörper (13) aus Silidonschaum besteht.

9. Vorricthung nach einem oder mehreren der Ansprüche 1 bis 5 oder 8, dadurch gekennzeichnet, daß der Abstrichkörper (13) von einem Vlies gebildet ist, dessen Struktur porenartige Aufnahmestellen für das Abstrichmaterial bildet.

10. Vorrichtung nach wenigstens einem der Ansprüche 1, 4, 6, 7, 8 oder 9, dadurch gekennzeichent, daß der Stiel (11) parallel zur Rückseite (R) des biegsamen Abstrichkörpers (13) od. dgl. Porenkörpers (14) sowie vorzugsweise etwa bis zu dessen Zentrum (Z) reichend an den Abstrichkörper (13) od. dgl. (14) befestigt ist.

11. Vorrichtung nach einem oder mehreren der Ansprüche 1 bis 10, dadurch gekennzeichnet, daß der Abstrichkörper (13) aus Polyurethan-Schaumstoff mittels Acrylat-Kleber am Stiel (11) befestigt ist.

## Fig.1

EP 0 307 792 A2

Fig.2

20   11   10   12   21  22  13
                        14
                        16
          D             15
          17            b

Fig.3   15
        16

Fig.4   20   10   18  16  15
20       d
20                      19
         L              13

Fig.5        10   13  15  18
20   11                 17

Fig.6        15a
             16

Fig.7   11   10a   12  17  13a
                   25
                   16

Fig.8

Fig.8a